# EUROPEAN PATENT SPECIFICATION

(11) **EP 3 980 107 B1**
(45) Date of publication and mention of the grant of the patent: **04.03.2026**
(21) Application number: 20747189.7
(22) Date of filing: 02.06.2020
(51) Int. Cl.: A61M 3/02, A61M 5/14, A61M 39/10, A61M 25/00, A61M 39/00

(54) **CATHETER ASSEMBLY FLUSHING DEVICE AND RELATED METHODS**
VORRICHTUNG ZUM SPÜLEN EINES KATHETERS UND ZUGEHÖRIGE VERFAHREN
DISPOSITIF DE RINÇAGE D'ENSEMBLE CATHÉTER ET PROCÉDÉS CONNEXES

(30) Priority: 05.06.2019 US 201962857360 P; 01.06.2020 US 202016889398
(43) Date of publication of application: 13.04.2022
(73) Proprietor: Becton, Dickinson and Company, Franklin Lakes, NJ 07417 (US)
(72) Inventor: WANG, Lionel, Suzhou Industry Park Jiangsu, Jiangsu 215021 (CN); XUE, Yueqiang, Shanghai 201203 (CN); DONG, Bin, Suzhou Industry Park Jainsu, Jiangsu 215021 (CN)
(74) Representative: dompatent
(86) International application number: PCT/US2020/035736
(87) International publication number: WO 2020/247384

(56) References cited:
- WO-A1-2009/147510
- WO-A1-2011/031864
- CN-Y- 2 186 577
- SE-B- 452 402
- SE-L- 8 601 122
- US-A1- 2003 181 824

## Description

### BACKGROUND

Catheters are generally used for parenteral nutrition, intravenous fluid replacement, and administering analgesics and antibiotics. Catheters are also used for blood draw. Catheters can be inserted at the bedside using sterile techniques and can remain in place for several weeks.

A common type catheter is an over-the-needle catheter. As its name implies, a catheter that is "over-the-needle" may be mounted over an introducer needle having a sharp distal tip. The sharp distal tip may be used to pierce skin and a vein of a patient. Insertion of the catheter into the vein may follow the piercing of the vein by the introducer needle. The introducer needle typically has the sharp distal tip to pierce skin and the vein of the patient with minimal resistance to minimize the pain to the patient.

The introducer needle is generally placed at a steep inclined angle with respect to a surface of the skin and a longitudinal dimension of the vein to be pierced to allow penetration through the skin and a wall of the vein. The needle and the catheter are generally inserted with a bevel of the introducer needle facing away from the skin of the patient. After the tip of the introducer needle pierces the wall, the angle of the insertion is lowered to be able to slide the introducer needle and the catheter into the vein a distance sufficient to properly position the catheter in the vein.

Once placement of the introducer needle within the vein has been confirmed, the user may temporarily occlude flow in the vein and withdraw the introducer needle, leaving the catheter in place for future fluid infusion and/or blood withdrawal. After blood withdrawal through the catheter, a catheter assembly, including the catheter, may be flushed to remove residual blood.

The subject matter claimed herein is not limited to embodiments that solve any disadvantages or that operate only in environments such as those described above. Rather, this background is only provided to illustrate one example technology area where some implementations described herein may be practiced.
WO 2009/147510 A1 discloses a catheter flush and fill unit.
US2003/0181824 A1 discloses a system for performing side specific therapy to alleviate tissue swelling.
WO 2011/031864 A1 discloses a method for simultaneously delivering fluid to a dual lumen catheter with a single fluid source.
SE8601122L discloses a filtering system for producing bacteria-free fluid.
CN 2186577Y discloses an intravenous infusion medicine feeding device.

### SUMMARY OF THE INVENTION

The subject matter of the invention is defined by independent claim 1.

The present disclosure relates generally to a device to flush a catheter assembly, as well as related systems and methods. In some embodiments, the device may include a first extension tube, which may include a distal end and a proximal end. In some embodiments, the device may include a first connector, which may be coupled to the distal end of the first extension tube. For example, the distal end of the first extension tube may be integrated with the first connector. In some embodiments, the device may include a second extension tube, which may include a distal end and a proximal end. In some embodiments, the device may include a second connector, which may be coupled to the distal end of the second extension tube. For example, the distal end of the second extension tube may be integrated with the second connector.

In some embodiments, the device may include a syringe, which may be in fluid communication with the first extension tube and the second extension tube. In some embodiments, the syringe may include a plunger. In some embodiments, a distal end of the syringe may include a luer connector.

In some embodiments, the device may include an extension, which may be coupled to the syringe. In some embodiments, a first fluid pathway may extend through the first extension tube, and a second fluid pathway may extend through the second extension tube. In some embodiments, a third fluid pathway may extend through the extension. In some embodiments, the third fluid pathway may bifurcate or branch into the first fluid pathway and the second fluid pathway. In some embodiments, the extension, the first extension tube, and the second extension tube may form a Y-shape, with each of the first extension tube and the second extension tube forming an arm of the Y-shape.

In some embodiments, a proximal end of the extension may include a third connector, which may be coupled to the luer connector of the syringe. In some embodiments, the extension may include a third extension tube. In some embodiments, a proximal end of the third extension tube may be coupled to the third connector, which may be coupled to the luer connector of the syringe. In some embodiments, the third connector may be directly coupled to the luer connector of the syringe. In some embodiments, the proximal end of the third extension tube may be integrated with the third connector.

In some embodiments, a first needleless connector may be coupled to the first connector and/or a second needleless connector may be coupled to the second connector. In some embodiments, the first needleless connector and the second needleless connector may be coupled to a first port and a second port, respectively, of an adapter of a catheter assembly. In some embodiments, the adapter of the catheter assembly may include a Y-adapter.

In some embodiments, the catheter assembly may also include a catheter adapter, which may include a distal end, a proximal end, and a lumen extending through the distal end and the proximal end. In some embodiments, the catheter assembly may include a catheter, which may extend distally from the distal end of the catheter adapter and may be secured within the catheter adapter. In some embodiments, the catheter adapter may include a fourth extension tube, which may extend from a side port of the catheter adapter. In some embodiments, a distal end of the fourth extension tube may be integrated within the side port. In some embodiments, a proximal end of the fourth extension tube may be coupled to the adapter, which may include at least the first port and the second port.

In some embodiments, a method of flushing the catheter assembly may include coupling the device to the adapter of the catheter assembly. In some embodiments, coupling the device to the adapter of the catheter assembly may include coupling the first extension tube to the first port of the adapter and coupling the second extension tube to the second port of the adapter. In some embodiments, coupling the device to the adapter of the catheter assembly may include coupling the first connector directly to the first port of the adapter and coupling the second connector directly to the second port of the adapter. In some embodiments, coupling the device to the adapter of the catheter assembly may include coupling the first connector directly to the first port of the adapter and coupling the second connector directly to the second port of the adapter. In some embodiments, coupling the device to the adapter of the catheter assembly may include coupling the first connector directly to the first needleless connector and coupling the second connector directly to the second needleless connector.

In some embodiments, the syringe may be filled with fluid. In some embodiments, the syringe may be filled with fluid prior to coupling of the device to the adapter and/or prior to coupling the syringe to the device. In some embodiments, the plunger of the syringe may be depressed, which may release fluid from the syringe.

In some embodiments, in response to depressing the plunger a single time, fluid within the syringe may flow through one or more of the following: the extension, the first extension tube, the second extension tube, the first connector, the second connector, the first needleless connector, the second needleless connector, the first port of the adapter, the second port of the adapter, and the catheter assembly. Thus, in some embodiments, in response to depressing the plunger the single time, fluid within the syringe may flush and clean one or more of the following: the first needleless connector, the second needleless connector, the first port of the adapter, the second port of the adapter, and the catheter assembly.

In some embodiments, the device may facilitate easy, efficient flushing in response to the single depression of the plunger. In some embodiments, the device may facilitate flushing of the first port and the second port simultaneously. In some embodiments, the device may facilitate flushing of the first needleless connector and the second needleless connector simultaneously. In some embodiments, the flushing may clean and remove blood within one or more of the first port, the second port, the first needleless connector, the second needleless connector, and the catheter assembly.

In some embodiments, the first extension tube and the second extension tube may be crossed prior to depressing the syringe. In some embodiments, crossing the first extension tube and the second extension tube may facilitate approximately equal flushing of the first port and the second port of the adapter. For example, the first port and the second port may be flushed with an approximately equal volume for an equal time in response to depressing the syringe the single time. In some embodiments, the first needleless connector and the second needleless connector may also be flushed with an approximately equal volume for an equal time in response to depressing the syringe the single time. In some embodiments, a length of the first extension tube and/or a length of the second extension tube may facilitate approximately equal flushing of the first port and the second port of the adapter. For example, the first extension tube may be longer than the second extension tube or the first extension tube and the second extension tube may be a same length.

In some embodiments, the first extension tube may be angled with respect to the second extension tube. In some embodiments, an angle between the first extension tube and the second extension tube may be between 0° and 90°. In some embodiments, the proximal end of the first extension tube and the proximal end of the second extension tube may be fixed at the angle. In some embodiments, the angle between the first extension tube and the second extension tube may be selected based on an angle between the first port and the second port of the adapter and may facilitate approximately equal flushing of the first port and the second port of the adapter and/or the first needleless connector and the second needleless connector.

It is to be understood that both the foregoing general description and the following detailed description are exemplary and explanatory and are not restrictive of the invention, as claimed. It should be understood that the various embodiments are not limited to the arrangements and instrumentality shown in the drawings. It should also be understood that the embodiments may be combined, or that other embodiments may be utilized and that structural changes, unless so claimed, may be made without departing from the scope of the various embodiments of the present invention. The following detailed description is, therefore, not to be taken in a limiting sense.

### BRIEF DESCRIPTION OF THE DRAWINGS

Example embodiments will be described and explained with additional specificity and detail through the use of the accompanying drawings in which:
Figure 1 is an upper perspective view of a prior art catheter system;
Figure 2A is an upper perspective view of a device to flush a catheter assembly, according to some embodiments;
Figure 2B is a cross-sectional view of the device of Figure 2A, illustrating a plunger of the device in a first position, according to some embodiments;
Figure 2C is a cross-sectional view of the device of the device of Figure 2A, illustrating the plunger of the device in a second position, according to some embodiments;
Figure 3A is an upper perspective view of a catheter system, according to some embodiments;
Figure 3B is a cross-sectional view of a portion of the catheter system, according to some embodiments; and
Figure 4 is a cross-sectional view of the catheter system, according to some embodiments.

### DESCRIPTION OF EMBODIMENTS

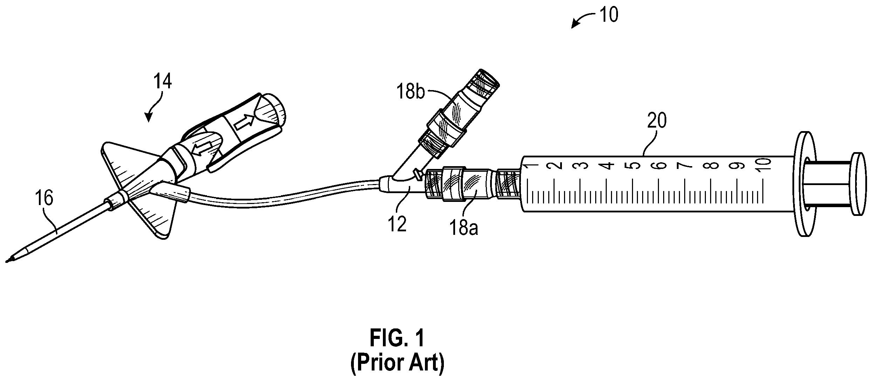

Referring now to Figure 1, a prior art catheter system 10 is illustrated. The prior art catheter system 10 includes a Y-adapter 12 in fluid communication with a catheter assembly 14. The catheter assembly 14 includes a catheter 16 that is configured to insert into vasculature of a patient. A first port and/or a second port of the Y-adapter 12 may be coupled to a needleless connector 18, such as, for example, a MaxPlus^{™} needleless connector, a MaxZero^{™} needleless connector, a BD Q-Syte^{™} luer activated split septum, a SmartSite^{™} needle-free connector available from Becton, Dickinson and Company (BD), or another suitable connector.

After drawing blood from the patient through the catheter assembly 14, residual blood may collect within one or more of the following: the first port, the second port, a first needleless connector 18a coupled to the first port, and a second needleless connector 18b coupled to the second port. In order to remove the blood from the catheter assembly 14 and clean the catheter assembly 14, the clinician may flush the catheter assembly 14. The prior art catheter system 10 does not facilitate simultaneous flushing of the first port and the second port. In order to flush the catheter assembly 14, the clinician may first couple a syringe 20 to the first port and activate the syringe 20, flushing the first port and/or the first needleless connector 18a. After the first port is flushed, the clinician may transfer the syringe 20 to the second port and activate the syringe 20 or activate another syringe coupled to the second port, flushing the second port and/or the second needleless connector 18b.

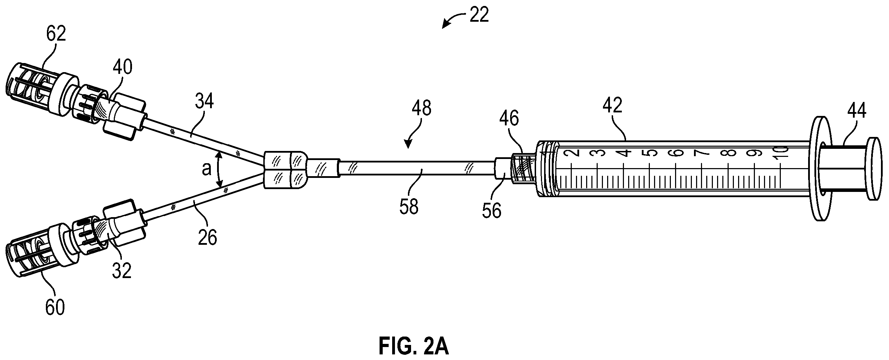

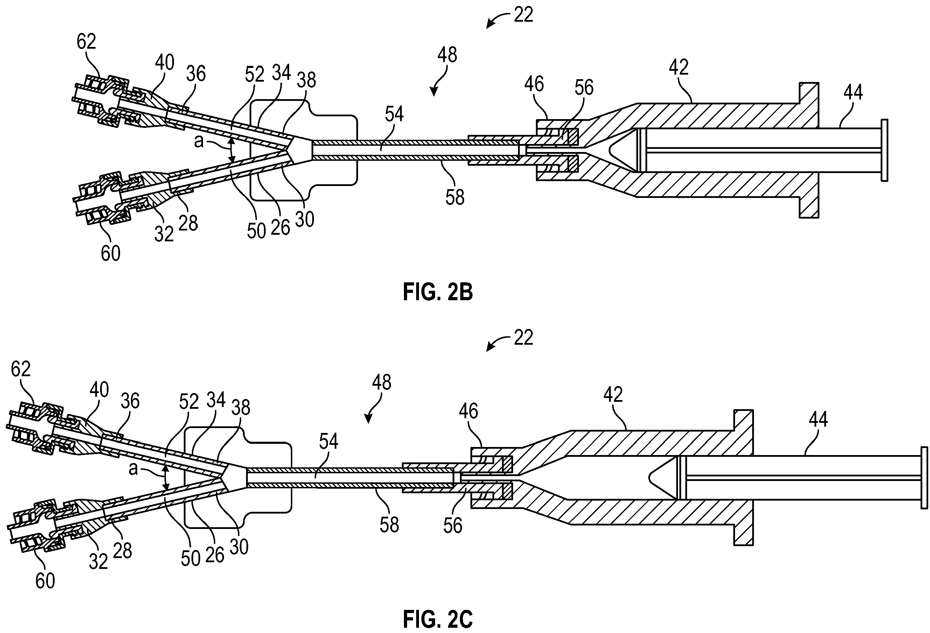

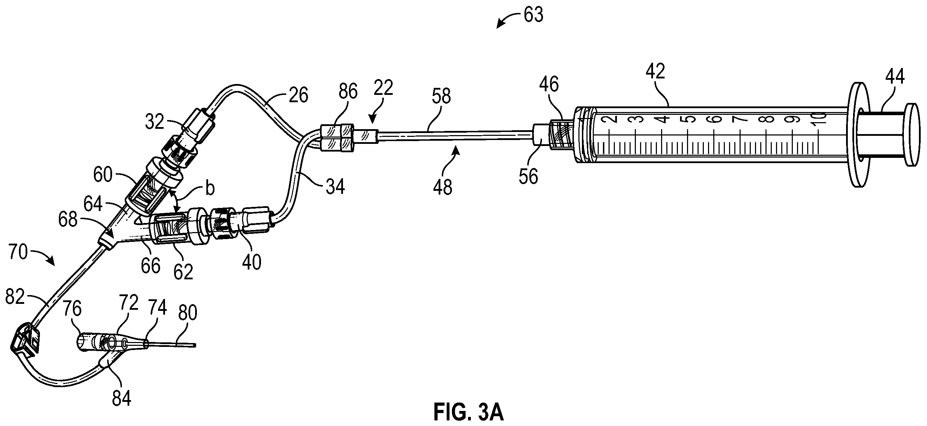

Referring now to Figures 2A-2C, a device 22 to flush a catheter assembly, such as, for example, the catheter assembly 14 illustrated in Figure 1 or the catheter assembly 70 illustrated in Figures 3A-3B, may include a first extension tube 26. In some embodiments, the first extension tube 26 may include a distal end 28 and a proximal end 30. In some embodiments, the device 22 may include a first connector 32, which may be coupled to the distal end 28 of the first extension tube 26. For example, the distal end 28 of the first extension tube 26 may be integrated with the first connector 32 as illustrated in Figures 2B-2C. In some embodiments, the first connector 32 and/or the second connector 40 may include a luer connector, such as, for example, a slip or thread male luer connector or a slip or thread female luer connector.

In some embodiments, the device 22 may include a second extension tube 34, which may include a distal end 36 and a proximal end 38. In some embodiments, the device 22 may include a second connector 40, which may be coupled to the distal end 36 of the second extension tube 34. For example, the distal end 36 of the second extension tube 34 may be integrated with the second connector 40 as illustrated in Figures 2B-2C.

In some embodiments, the device 22 may include a syringe 42, which may be in fluid communication with the first extension tube 26 and the second extension tube 34. In some embodiments, the syringe 42 may include a plunger 44. In some embodiments, a distal end of the syringe 42 may include a luer connector 46, such as, for example, a slip or thread male luer connector or a slip or thread female luer connector. In some embodiments, the syringe 42 may include any suitable syringe.

In some embodiments, the device may include an extension 48, which may be coupled to the syringe 42. In some embodiments, a first fluid pathway 50 may extend through the first extension tube 26, and a second fluid pathway 52 may extend through the second extension tube 34. In some embodiments, a third fluid pathway 54 may extend through the extension 48. In some embodiments, the third fluid pathway 54 may bifurcate or branch into the first fluid pathway 50 and the second fluid pathway 52. In some embodiments, the extension 48, the first extension tube 26, and the second extension tube 34 may form a Y-shape. In some embodiments, the third fluid pathway 54 may branch into the first fluid pathway 50, the second fluid pathway 52, and one or more other fluid pathways, which may extend through one or more other extension tubes (not illustrated).

In some embodiments, a proximal end of the extension 48 may include a third connector 56, which may be coupled to the syringe 42, such as the luer connector 46 of the syringe 42. In some embodiments, the third connector 56 may include a luer connector, such as, for example, a slip or thread male luer connector or a slip or thread female luer connector. In some embodiments, the extension 48 may include a third extension tube 58. In some embodiments, a proximal end of the third extension tube 58 may be coupled to the third connector 56, which may be coupled to the luer connector 46 of the syringe 42. In some embodiments, the proximal end of the third extension tube 58 may be integrated with the third connector 56 as illustrated in Figures 2B-2C. In some embodiments, the syringe 42 may be removably coupled to the third connector 56, which may allow the syringe 42 to be filled with fluid and then coupled to the third connector 56.

In some embodiments, a first needleless connector 60 may be coupled to the first connector 32 and/or a second needleless connector 62 may be coupled to the second connector 40. In some embodiments, the extension 48 may include the third extension tube 58 and/or one or more other components. In some embodiments, the one or more other components may include connectors, extension tubes, housings, etc. In some embodiments, the extension 48 may be monolithically formed as a single unit or may multiple components, which may be coupled together. In some embodiments, the extension 48 may include any suitable component or combination of components that includes the third fluid pathway 54 extending there through and is disposed between the syringe 42 and the first extension tube 26 and between the syringe 42 and the second extension tube 34. In some embodiments, the device 22 may not include the extension 48. In some embodiments, the first extension tube 26 and the second extension tube 34 may extend directly from the syringe 20.

In some embodiments, the first extension tube 26 may be angled with respect to the second extension tube 34. In some embodiments, an angle *a* between the first extension tube 26 and the second extension tube 34 may be between 0° and 90°. In some embodiments, the angle *a* may be between 30° and 60°. In some embodiments, the angle *a* may be between 15° and 45°. In some embodiments, the proximal end 30 of the first extension tube 26 and the proximal end 38 of the second extension tube 34 may be fixed at the angle *a.* In some embodiments, proximal end 30 of the first extension tube 26 and the proximal end 38 the second extension tube 34 may be parallel and the angle *a* may be 0°. In some embodiments, the angle *a* between the first extension tube 26 and the second extension tube 34 may be selected based on an angle *b* (see, for example, Figure 3A) between a first port and a second port of the adapter 68 and may facilitate approximately equal flushing of the first port and the second port of the adapter 68 and/or the first needleless connector 60 and the second needleless connector 62.

Referring now to Figures 3A-3B, a catheter system 63 is illustrated, according to some embodiments. In some embodiments, a distal end of the first needleless connector 60 and a distal end of the second needleless connector 62 may be coupled to a first port 64 and a second port 66, respectively, of an adapter 68 of a catheter assembly 70 of the catheter system 63. In some embodiments, the adapter 68 of the catheter assembly may include a Y-adapter. In some embodiments, the first connector 32 may be directly coupled to the first port 64 and/or the second connector 40 may be directly coupled to the second port 66. In these and other embodiments, the catheter system 63 may not include the first needleless connector 60 and/or the second needleless connector 62.

In some embodiments, the catheter assembly 70 may include a catheter adapter 72, which may include a distal end 74, a proximal end 76, and a lumen 78 extending through the distal end 74 and the proximal end 76. In some embodiments, the catheter assembly 70 may include a catheter 80, which may extend distally from the distal end 74 of the catheter adapter 72 and may be secured within the catheter adapter 72. In some embodiments, the catheter adapter 72 may include a fourth extension tube 82, which may extend from a side port 84 of the catheter adapter 72. In some embodiments, a distal end of the fourth extension tube 82 may be integrated within the side port 84. In some embodiments, a proximal end of the fourth extension tube 82 may be coupled to the adapter 68, which may include at least the first port 64 and the second port 66.

In some embodiments, flushing the catheter assembly 70 may include coupling the device 22 to the adapter 68 of the catheter assembly 70. In some embodiments, coupling the device 22 to the adapter 68 of the catheter assembly 70 may include coupling the first extension tube 26 to the first port 64 of the adapter 68 and coupling the second extension tube 34 to the second port 66 of the adapter 68. In some embodiments, coupling the device 22 to the adapter 68 of the catheter assembly 70 may include coupling the first connector 32 directly to the first port 64 of the adapter 68 and coupling the second connector 40 directly to the second port 66 of the adapter 68. In some embodiments, coupling the device 22 to the adapter 68 of the catheter assembly 70 may include coupling the first connector 32 directly to the first port 64 of the adapter 68 and coupling the second connector 40 directly to the second port 66 of the adapter 68. In some embodiments, coupling the device 22 to the adapter 68 of the catheter assembly 70 may include coupling the first connector 32 directly to the first needleless connector 60 and coupling the second connector 40 directly to the second needleless connector 62.

In some embodiments, the second extension tube 34 may be coupled to the second port and/or the second needleless connector 62 when the first extension tube 26 is coupled to the first port 64 such that fluid may simultaneously flow from the first extension tube 26 to the catheter assembly 70 and from the second extension tube 34 to the catheter assembly 70. In some embodiments, the syringe 42 may be filled with fluid. In some embodiments, the plunger 44 of the syringe 42 may be depressed to activate the syringe 42, which may release fluid from the syringe 42.

In some embodiments, in response to depressing the plunger 44 a single time, fluid within the syringe 42 may flow through one or more of the following: the extension 48, the first extension tube 26, the second extension tube 34. the first connector 32, the second connector 40, the first needleless connector 60, the second needleless connector 62, the first port 64 of the adapter 68, the second port 66 of the adapter 68, and the catheter assembly 70. Thus, in some embodiments, in response to depressing the plunger 44 the single time, fluid within the syringe 42 may flush and clean one or more of the following: the first needleless connector 60, the second needleless connector 62, the first port 64 of the adapter 68, the second port 66 of the adapter 68, and the catheter assembly 70. In some embodiments, the device 22 may facilitate easy, efficient flushing in response to the single depression of the plunger 44. In some embodiments, the device 22 may facilitate flushing of the first port 64 and the second port 66 simultaneously. In some embodiments, the device 22 may facilitate flushing of the first needleless connector 60 and the second needleless connector 62 simultaneously.

In some embodiments, the first extension tube 26 and the second extension tube 34 may be crossed prior to depressing the syringe 42. In some embodiments, crossing the first extension tube 26 and the second extension tube 34 may facilitate approximately equal flushing of the first port 64 and the second port 66 of the adapter 68. For example, the first port 64 and the second port 66 may be flushed with an approximately equal volume for an equal time in response to depressing the syringe 42 the single time. In some embodiments, the first needleless connector 60 and the second needleless connector 62 may also be flushed with an approximately equal volume for an equal time in response to depressing the syringe 42 the single time.

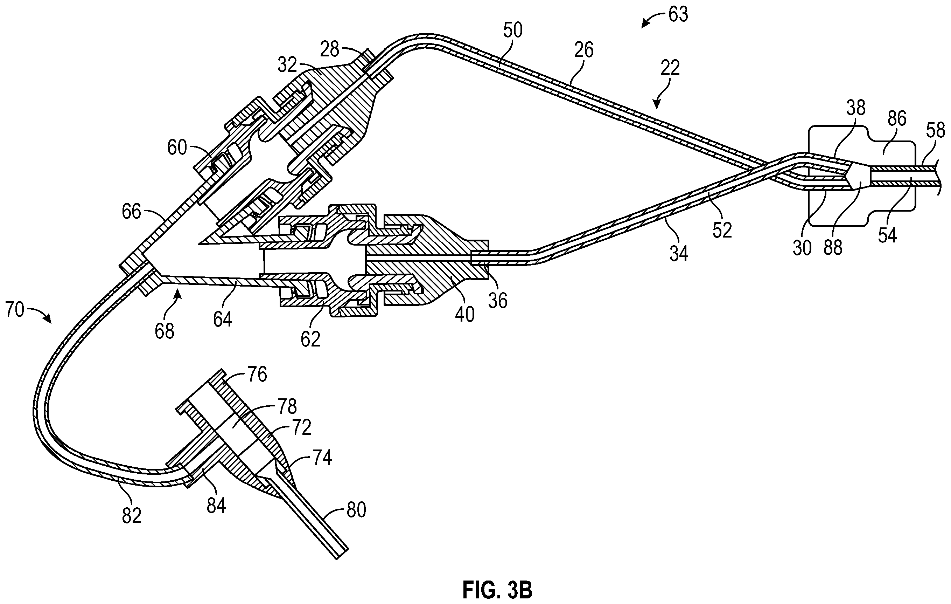

In some embodiments, the proximal end 30 of the first extension tube 26 and/or the proximal end 38 of the second extension tube 34 may be fixed within a housing 86. In some embodiments, a distal end of the third extension tube 58 or a distal end of the extension 48 may be removably coupled to the housing 86 via a connector, for example. In some embodiments, as illustrated, for example, in Figure 3B, the third extension tube 58 may be integrated within the housing 86. In some embodiments, a space 88 within the housing 86 may join the third fluid pathway 54 with the first and second fluid pathways 50, 52.

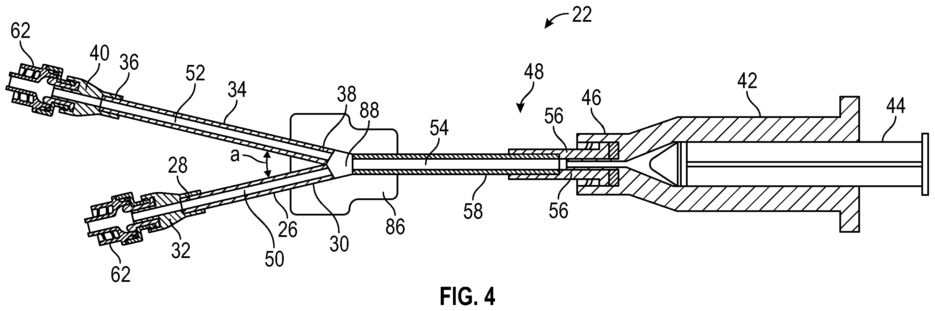

Referring now to Figure 4, in some embodiments, a length of the first extension tube 26 and/or a length of the second extension tube 34 may facilitate approximately equal flushing of the first port 64 and the second port 66 of the adapter 68. For example, the first extension tube 26 may be longer than the second extension tube 34 or the first extension tube 26 and the second extension tube 34 may be a same length. In some embodiments, the length of the first extension tube 26 with respect to the second extension tube 34 may be selected based on a shape of the adapter 68.

All examples and conditional language recited herein are intended for pedagogical objects to aid the reader in understanding the invention and the concepts contributed by the inventor to furthering the art, and are to be construed as being without limitation to such specifically recited examples and conditions. Although embodiments of the present inventions have been described in detail, it should be understood that the various changes, substitutions, and alterations could be made hereto without departing from the scope of the invantion as defined by the claims.

## Claims

1. A device to flush a catheter assembly (14), the device comprising:
a first extension tube (26), comprising a distal end (28), a proximal end (30) , and a first fluid pathway (50) extending through the first extension tube;
a first connector (32) coupled to the distal end of the first extension tube;
a second extension tube (34), comprising a distal end (36), a proximal end (38) , and a second fluid pathway (52) extending through the second extension tube;
a second connector (40) coupled to the distal end of the second extension tube;
a housing (86), wherein the proximal end of the first extension tube and the proximal end of the second extension tube are fixed within the housing;
a syringe (42) in fluid communication with the first extension tube and the second extension tube, a first needleless connector (60) coupled to the first connector; and
a second needleless connector (62) coupled to the second connector,
**characterized in that**
the first extension tube is longer than the second extension tube, and in response to crossing the first and second extension tubes and depressing the syringe a single time, the first fluid pathway, the second fluid pathway, the first connector, and the second connector are each flushed with an approximately equal volume of fluid for an equal time.

2. The device of claim 1, further comprising:
an extension (48) coupled to the syringe and the housing, wherein the extension comprises a third extension tube (58) comprising;
a third fluid pathway (54) extending through the third extension, wherein the third fluid pathway branches into the first fluid pathway and the second fluid pathway.

3. The device of claim 2, wherein a distal end of the syringe comprises a luer connector (46), wherein a proximal end of the extension comprises a third connector (56) coupled to the luer connector.

4. The device of claim 2, wherein the third extension tube comprises a distal end and a proximal end, wherein the proximal end of the third extension tube is coupled to a third connector (56).

5. The device of claim 1, wherein the proximal end of the first extension tube is disposed at a fixed angle with respect to the proximal end of the second extension tube.

6. The device of claim 5, wherein an angle between the first extension tube and the second extension tube is between 30° and 60° or between 15° and 45°.

7. A method of flushing a catheter assembly (14), the method comprising:
coupling an adapter (12) of the catheter assembly to a device (22) according to any of claims 1 to 6; and
depressing the syringe a single time,
**characterized in that**
in response to depressing the syringe the single time, the first fluid pathway, the second fluid pathway, the first connector, and the second connector are each flushed with an approximately equal volume of a fluid for an equal time.

8. The method of claim 7, wherein the adapter (12) includes a Y-adapter.

## Patentansprüche

1. Vorrichtung zum Spülen einer Katheterbaugruppe (14), wobei die Vorrichtung aufweist:
einen ersten Verlängerungsschlauch (26), der ein distales Ende (28), ein proximales Ende (30) und einen ersten Fluidweg (50) aufweist, der sich durch den ersten Verlängerungsschlauch erstreckt;
einen ersten Konnektor (32), der mit dem distalen Ende des ersten Verlängerungsschlauchs gekoppelt ist;
einen zweiten Verlängerungsschlauch (34), der ein distales Ende (36), ein proximales Ende (38) und einen zweiten Fluidweg (52) aufweist, der sich durch den zweiten Verlängerungsschlauch erstreckt;
einen zweiten Konnektor (40), der mit dem distalen Ende des zweiten Verlängerungsschlauchs gekoppelt ist;
ein Gehäuse (86), wobei das proximale Ende des ersten Verlängerungsschlauchs und das proximale Ende des zweiten Verlängerungsschlauchs in dem Gehäuse befestigt sind;
eine Spritze (42), die in Fluidverbindung mit dem ersten Verlängerungsschlauch und dem zweiten Verlängerungsschlauch steht, einen ersten nadellosen Konnektor (60), der mit dem ersten Konnektor gekoppelt ist; und
einen zweiten nadellosen Konnektor (62), der mit dem zweiten Konnektor gekoppelt ist,
**dadurch gekennzeichnet, dass**
der erste Verlängerungsschlauch länger ist als der zweite Verlängerungsschlauch, und als Reaktion auf das Kreuzen des ersten und des zweiten Verlängerungsschlauchs und das einmalige Niederdrücken der Spritze der erste Fluidweg, der zweite Fluidweg, der erste Konnektor und der zweite Konnektor jeweils mit einem ungefähr gleichen Fluidvolumen eine gleiche Zeit lang gespült werden.

2. Vorrichtung nach Anspruch 1, die ferner aufweist:
eine Verlängerung (48), die mit der Spritze und dem Gehäuse verbunden ist, wobei die Verlängerung einen dritten Verlängerungsschlauch (58) aufweist;
einen dritten Fluidweg (54), der sich durch die dritte Verlängerung erstreckt, wobei sich der dritte Fluidweg in den ersten Fluidweg und den zweiten Fluidweg verzweigt.

3. Vorrichtung nach Anspruch 2, wobei ein distales Ende der Spritze einen Luer-Anschluss (46) aufweist, wobei ein proximales Ende der Verlängerung einen dritten Konnektor (56) aufweist, der mit dem Luer-Anschluss gekoppelt ist.

4. Vorrichtung nach Anspruch 2, wobei der dritte Verlängerungsschlauch ein distales Ende und ein proximales Ende aufweist, wobei das proximale Ende des dritten Verlängerungsschlauchs mit einem dritten Konnektor (56) gekoppelt ist.

5. Vorrichtung nach Anspruch 1, wobei das proximale Ende des ersten Verlängerungsschlauchs in einem festen Winkel in Bezug auf das proximale Ende des zweiten Verlängerungsschlauchs angeordnet ist.

6. Vorrichtung nach Anspruch 5, wobei der Winkel zwischen dem ersten Verlängerungsschlauch und dem zweiten Verlängerungsschlauch zwischen 30° und 60° oder zwischen 15° und 45° liegt.

7. Verfahren zum Spülen einer Katheterbaugruppe (14), wobei das Verfahren umfasst:
Koppeln eines Adapters (12) der Katheterbaugruppe mit einer Vorrichtung (22) nach einem der Ansprüche 1 bis 6; und
einmaliges Niederdrücken der Spritze,
**dadurch gekennzeichnet, dass**
als Reaktion auf das einmalige Niederdrücken der Spritze der erste Fluidweg, der zweite Fluidweg, der erste Konnektor und der zweite Konnektor jeweils mit einem ungefähr gleichen Fluidvolumen eine gleiche Zeit lang gespült werden.

8. Verfahren nach Anspruch 7, wobei der Adapter (12) einen Y-Adapter umfasst.

## Revendications

1. Dispositif pour rincer un ensemble cathéter (14), le dispositif comprenant :
un premier tube d'extension (26) avec une extrémité distale (28), une extrémité proximale (30) et un premier chemin de passage pour fluide (50) s'étendant par le premier tube d'extension ;
un premier connecteur (32) accouplé à l'extrémité distale du premier tube d'extension ;
un deuxième tube d'extension (34) avec une extrémité distale (36), une extrémité proximale (38) et un deuxième chemin de passage pour fluide (52) s'étendant par le deuxième tube d'extension ;
un deuxième connecteur (40) accouplé à l'extrémité distale du deuxième tube d'extension ;
un boîtier (86), l'extrémité proximale du premier tube d'extension et l'extrémité proximale du deuxième tube d'extension étant montées à l'intérieur du boîtier ;
une seringue (42) en communication fluidique avec le premier tube d'extension et le deuxième tube d'extension, un premier connecteur sans aiguille (60) accouplé au premier connecteur, et
un deuxième connecteur sans aiguille (62) accouplé au deuxième connecteur,
**caractérisé en ce que**
le premier tube d'extension est plus long que le deuxième tube d'extension et, en réponse à un croisement des premier et deuxième tubes d'extension et à une dépression de la seringue en une seule fois, le premier chemin de passage pour fluide, le deuxième chemin de passage pour fluide, le premier connecteur et le deuxième connecteur sont rincés chacun avec un volume approximativement égal de fluide pendant un temps égal.

2. Dispositif selon la revendication 1, comprenant en outre :
une extension (48) accouplée à la seringue et le boîtier, l'extension comprenant un troisième tube d'extension (58) avec :
un troisième chemin de passage pour fluide (54) s'étendant par la troisième extension, le troisième chemin de passage pour fluide étant divisé en le premier chemin de passage pour fluide et le deuxième chemin de passage pour fluide.

3. Dispositif selon la revendication 2, une extrémité distale de la seringue comprenant un connecteur Luer (46), une extrémité proximale de l'extension comprenant un troisième connecteur (56) accouplée au connecteur Luer.

4. Dispositif selon la revendication 2, le troisième tube d'extension comprenant une extrémité distale et une extrémité proximale, l'extrémité proximale du troisième tube d'extension étant accouplée à un troisième connecteur (56).

5. Dispositif selon la revendication 1, l'extrémité proximale du premier tube d'extension étant disposée sous un angle fixe par rapport à l'extrémité proximale du deuxième tube d'extension.

6. Dispositif selon la revendication 5, un angle entre le premier tube d'extension et le deuxième tube d'extension étant entre 30° et 60° ou entre 15° et 45°.

7. Procédé de rinçage d'un ensemble cathéter (14), le procédé comprenant :
accoupler un adaptateur (12) de l'ensemble cathéter à un dispositif (22) selon l'une des revendications 1 à 6, et
dépresser la seringue en une seule fois,
**caractérisé en ce que**
en réponse à la dépression de la seringue en une seule fois, le premier chemin de passage pour fluide, le deuxième chemin de passage pour fluide, le premier connecteur et le deuxième connecteur sont rincés chacun avec un volume approximativement égal de fluide pendant un temps égal.

8. Procédé selon la revendication 7, l'adaptateur (12) comprenant un adaptateur en Y.
